# EUROPEAN PATENT APPLICATION

(11) **EP 2 620 140 A1**
(43) Date of publication of application: **31.07.2013**
(21) Application number: 12000519.4
(22) Date of filing: 26.01.2012
(51) Int. Cl.: A61K 9/20, A61K 31/4439

(54) **Crizotinib containing compositions**

(71) Applicant: ratiopharm GmbH, 89079 Ulm (DE)
(72) Inventor: Janssen, Christian, 89171 Illerkirchberg (DE); Prohl, Sabine, 81379 München (DE); Stefan, Ralph, 88370 Ebenweiler (DE)
(74) Representative: Aechter, Bernd

(57) **Abstract**

The present invention relates to a pharmaceutical composition containing non-crystalline crizotinib together with a surface stabiliser. The invention further relates to methods of preparing stable pharmaceutical compositions containing non-crystalline crizotinib. Further, the invention relates to an oral dosage form containing the above pharmaceutical composition and to methods of producing said oral dosage form.

## Description

### Background of the Invention

The present invention relates to a pharmaceutical composition containing non-crystalline crizotinib and a surface stabiliser. The invention further relates to methods of preparing said pharmaceutical compositions and to oral dosage forms containing the above pharmaceutical compositions.

"Crizotinib" is reported to be the INN name of 3-[(1R)-1-(2,6-dichloro-3-fluorophenyl)ethoxy]-5-(1-piperidin-4-ylpyrazol-4-yl)pyridin-2-amine and is characterized by the following chemical formula(I):

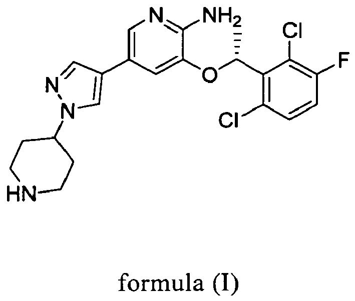

Crizotinib is reported to belong to the class of kinase inhibitors. Specifically, activity against the anaplastic lymphoma kinase (ALK) is reported, which can play different roles in oncogenesis.

EP 1 786 785 describes the synthesis of the compound crizotinib. EP 1 963 302 describes an apparently crystalline form of the free base which is designated Form 1.

However, it turned out that crizotinib, as known in the art, shows low solubility and permeability, so it is presumably classified as a class IV agent according to the FDA Biopharmaceutics Classification System (BCS). Therefore, it is a problem to obtain crizotinib-containing formulations with sufficient bioavailability, a linear pharmacokinetic, stability as well as content uniformity.

Hence, it was an object of the present invention to overcome these problems and to provide crizotinib in a form having superior absorption in the intestine and thus, superior bioavailability, preferably in combination with good stability and storage ability. In particular, a storage stability at 40 °C and 75% humidity for 12 months should be achieved. After storage under said conditions, impurities should be less than 2 wt.%, more preferably less than 1 wt.%. Moreover, it was an object to provide a dosage form having essentially a linear pharmacokinetic.

In addition, crizotinib should be provided in a form, which allows oral application. Further, the increase in permeability and bioavailability should be achieved without negatively influencing the flowability properties during production.

Furthermore, it was an object of the invention to provide crizotinib in a form, which possesses good processing abilities, for example flowability. In addition, it is intended to enable even distribution of the active pharmaceutical agent because of the great importance of the content conformity.

### Summary of the Invention

According to the present invention, the above objectives are achieved by specific pharmaceutical compositions comprising crizotinib and a surface stabilizer described herein. Said pharmaceutical compositions further can be used for processing oral dosage forms containing crizotinib in a non-crystalline form. Furthermore, the above drawbacks can be overcome by processes for producing said pharmaceutical compositions comprising crizotinib and a surface stabilizer and assuring that crizotinib is obtained in a non-crystalline form, for example by means of a melt process.

Thus, the subject of the present invention is a pharmaceutical composition containing non-crystalline crizotinib (a) in a stabilized form. In particular, the subject matter of the invention is a pharmaceutical composition comprising non-crystalline crizotinib (a) and surface stabiliser (b).

It was found that the pharmaceutical composition of the present invention has an enhanced bioavailability and superior permeability, resulting in a superior plasma profiles of the active pharmaceutical ingredient. Further, an improved content uniformity of the drug can be achieved, which can ensure that the appropriate dose can be applied to the patient. Even further it was found that the pharmaceutical composition of the present invention can be very stable over a long period.

The subject matter of the invention also relates to various methods of preparing non-crystalline crizotinib (a) in the form of the pharmaceutical composition of the invention.

Another subject of the present invention is an oral dosage form comprising the pharmaceutical composition of the invention. A further subject is a process of producing an oral dosage from, comprising the steps of
(11) providing crizotinib and surface stabilizer (b) and optionally one or more excipient(s) (c),
(12) processing the mixture of step (11) to a pharmaceutical composition, in which non-crystalline crizotinib is obtained,
(13) optionally granulating the pharmaceutical composition of step (12),
(14) mixing the pharmaceutical composition from step (12) or the granules from step (13) with optionally one or more excipient(s) (c'), and
(15) optionally processing the mixture of step (14) into an oral dosage form.

### Detailed description of the invention

In the context of this invention, the term "crizotinib" usually refers to 3-[(1R)-1-(2,6-dichloro-3-fluorophenyl) ethoxy]-5-(1-piperidin-4-ylpyrazol-4-yl)pyridin-2-amine in accordance with formula (1) above. In addition, the term "crizotinib" as used in the present application can refer to crizotinib in the form of the free base as well as to its pharmaceutically acceptable salts, hydrates, solvates, polymorphs and mixtures thereof. The pharmaceutical acceptable salts can be obtained by the reaction of crizotinib with an acid.

In a preferred embodiment of the present invention crizotinib is used in the form of the free base.

Generally, the term "non-crystalline" refers to any solid state being non-crystalline. Preferably, non-crystalline crizotinib means amorphous crizotinib, crizotinib in form of a solid dispersion or solid solution. Particularly preferred is amorphous crizotinib as compound (a).

The term "amorphous" can be used in the context of this invention to designate the state of solid substances, in which the components (atoms, ions or molecules, i.e. in the case of amorphous crizotinib the crizotinib molecules) do not exhibit any periodic arrangement over a great range (= long-range order). In amorphous substances, the components are usually not arranged in a totally disordered fashion and completely randomly, but are rather distributed in such a way that a certain regularity and similarity to the crystalline state can be observed with regard to the distance from and orientation towards their closest neighbours (= short-range order). Amorphous substances consequently preferably possess a short-range order, but no long-range order.

In contrast to anisotropic crystals, solid non-crystalline substances can be isotropic. Normally, they do not have a defined melting point, but instead pass over into the liquid state after slowly softening. They can be distinguished from crystalline substances experimentally by means of X-ray diffraction, which does not reveal clearly defined interferences for them, but rather, in most cases, only a few diffuse interferences with small diffraction angles.

The non-crystalline crizotinib (a) in the pharmaceutical composition of the invention may consist of purely non-crystalline crizotinib (a). Alternatively, it may also contain small amounts of crystalline crizotinib components, provided that no defined melting point of crystalline crizotinib can be detected in a DSC. A mixture containing 60 to 99.999% by weight non-crystalline crizotinib (a) and 0.001 to 40% by weight crystalline crizotinib is preferred, more preferably 90 to 99.99% by weight non-crystalline crizotinib (a) and 0.01 to 10% crystalline crizotinib, particularly preferably 95 to 99.9% by weight non-crystalline crizotinib (a) and 0.1 to 5% crystalline crizotinib.

Further, the pharmaceutical compositions of the invention are obtainable by a variety of preparation methods. Depending on the preparation method, the pharmaceutical compositions of the present invention, preferably the pharmaceutical composition consisting of compounds (a) and (b), are obtained in different particle sizes. Normally, the pharmaceutical compositions of the invention, preferably the pharmaceutical composition consisting of compounds (a) and (b), are present in particulate form and have an average particle size (D50) of 5 to 500 µm, preferably 20 to 300 µm, more preferably 55 to 190 µm.

The average particle size can refer to the D50-value of the particle size distribution. The average particle can be determined by means of laser diffractometry. In particular, a Malvern Instruments Mastersizer 2000 can be used to determine the size (preferably wet measurement with ultrasound 60 sec., 2,000 rpm, preferably dispersed in liquid paraffin, the evaluation being performed according to the Fraunhofer model).

The average particle size (D50), which is also denoted D50-value of the integral volume distribution, is defined in the context of this invention as the particle diameter, at which 50 percent by volume of the particles have a smaller diameter than the diameter which corresponds to the D50-value. Likewise, 50 percent by volume of the particles have a larger diameter than the D50-value. Analogous, the D90-value of the integral volume distribution is defined as the particle diameter, at which 90 percent by volume of the particles have a smaller diameter than the diameter which corresponds to the D90-value. Correspondingly, the D10-value of the integral volume distribution is defined as the particle diameter, at which 10 percent by volume of the particles have a smaller diameter than the diameter which corresponds to the D10-value.

In a particular preferred embodiment the pharmaceutical composition of the invention comprise crizotinib as sole pharmaceutical active agent.

In another preferred embodiment the pharmaceutical composition of the invention can comprise crizotinib in combination with further pharmaceutical active agent(s).

Preferably, the pharmaceutical composition of the present invention comprises 50 to 1000 mg crizotinib, more preferably 75 to 750 mg crizotinib, still more preferably 100 to 500 mg crizotinib, particularly 150 to 300 mg crizotinib. The amounts generally refer to crizotinib in form of the free base (i.e. if crizotinib is present in form of a salt, the amount of the salt has to be added accordingly).

In a preferred embodiment, the crizotinib in the pharmaceutical composition of the present invention can be present in stabilised form, namely in the form of a pharmaceutical composition containing non-crystalline crizotinib (a) and a surface stabiliser (b).

In a preferred embodiment of the invention the pharmaceutical composition can comprise crizotinib (a) and surface stabiliser (b), in which the weight ratio of non-crystalline crizotinib (a) to surface stabiliser (b) can be from 1: 20 to 20:1, preferably from 1:15 to 10:1, more preferably form 1:10 to 5:1 and particularly from 1:3 to 2:1.

The surface stabiliser (b) is a substance, which can usually inhibit the recrystallisation of non-crystalline to crystalline crizotinib. In a preferred embodiment the surface stabiliser (b) is a substance, inhibiting the recrystallisation of non-crystalline to crystalline crizotinib. Preferably, the recrystallisation is inhibited such that not more than 20%, preferably not more than 10% of the non-crystalline crizotinib present at the time of manufacture (T0) is transformed into crystalline crizotinib upon storage for three months at 45 °C / 75% relative humidity (RH).

The surface stabiliser (b) can preferably be a polymer. In addition, the surface stabiliser (b) can also include substances which behave like polymers. Examples of these substances are fats and waxes. Furthermore, the surface stabiliser (b) can also include solid, non-polymeric compounds which preferably can contain polar side groups. Examples of these compounds are sugar alcohols or disaccharides.

In a preferred embodiment the surface stabiliser (b) can be a polymer. The polymer to be used for the preparation of the pharmaceutical composition preferably may have a glass transition temperature (Tg) of more than 25 °C, more preferably 40 °C to 150 °C, in particular from 50 °C to 120 °C. During immobilisation a polymer with a Tg selected accordingly can be particularly advantageous in preventing the recrystallisation of the non-crystalline crizotinib (a).

The term "glass transition temperature" (Tg) is reported to describe the temperature at which amorphous or partially crystalline polymers change from the solid state to the liquid state. In the process, a distinct change in physical parameters, e.g. hardness and elasticity, occurs. Beneath the Tg, a polymer is usually glassy and hard, whereas above the Tg, it changes into a rubber-like to viscous state. The glass transition temperature is determined in the context of this invention by means of dynamic differential scanning calorimetry (DSC).

For this purpose, a Mettler Toledo DSC 1 apparatus can be used. The work is performed at a heating rate of 1-20 °C/min, preferably 5-15 °C/min, and at a cooling rate of 5-25 °C/min, preferably 10-20 °C/min.

In addition, the polymer to be used for the preparation of the pharmaceutical composition preferably can have a weight-average molecular weight of 1,000 to 500,000 g/mol, more preferably from 1,500 to 100,000 g/mol and particularly from 2,000 to 50,000 g/mol. The weight-average molecular weight is determined by means of gel permeation chromatography.

If the polymer used for the preparation of the pharmaceutical composition is dissolved in water in an amount of 2% by weight, the resulting solution preferably can have a viscosity of 1 to 20 mPa•s, more preferably 1,5 to 15 mPa•s, and even more preferably from 2 to 8 mPa•s or (especially in the case of HPMC) from 12 to 18 mPa•s, measured at 25 °C, and determined in accordance with Ph. Eur. 6.0, chapter 2.2.10.

Hydrophilic polymers can preferably be used for the preparation of the pharmaceutical composition of the present application. This can refer to polymers which possess hydrophilic groups. Examples of suitable hydrophilic groups can be hydroxy, sulfonate, carboxylate and quaternary ammonium groups.

The pharmaceutical composition of the invention may, for example, comprise the following polymers: polysaccharides, such as hydroxypropyl methyl cellulose (HPMC), carboxymethyl cellulose (CMC, especially sodium and calcium salts), ethyl cellulose, methyl cellulose, hydroxyethyl cellulose, ethyl hydroxyethyl cellulose, hydroxypropyl cellulose (HPC) hydroxypropyl methyl cellulose acetate succinate (HPMCAS), hydroxypropyl methyl cellulose (HPMCS), hydroxypropyl cellulose acetate succinate (HPCAS), hydroxyethyl methyl cellulose succinate (HEMCS), hydroxyethyl cellulose acetate succinate (HECAS), hydroxypropyl methyl cellulose phthalate (HPMCP), hydroxyethyl methyl cellulose acetate succinate (HEMCAS), carboxymethyl cellulose (CMC); polyvinylpyrrolidone, polyvinyl alcohol, polymers of acrylic acid and their salts, vinyl pyrrolidone-vinyl acetate copolymers (such as Kollidon^{®} VA 64, BASF), gelatine polyalkylene glycols, such as polypropylene glycol or preferably polyethylene glycol, gelatine and mixtures thereof.

Likewise, it can preferably be possible to use sugar alcohols such as mannitol, sorbitol, xylitol as surface stabilisers.

The surface stabiliser (b) preferably used can be polyvinylpyrrolidone, preferably with a weight-average molecular weight of 10,000 to 60,000 g/mol, especially 12,000 to 40,000 g/mol, vinylpyrrolidone and vinyl acetate copolymer, especially with a weight-average molecular weight of 45,000 to 75,000 g/mol and/or polymers of acrylic acid and their salts, especially with a weight-average molecular weight of 50,000 to 250,000 g/mol. In addition, HPMC can be preferably used, especially with a weight-average molecular weight of 20,000 to 90,000 g/mol and/or preferably a proportion of methyl groups of 10 to 35% and a proportion of hydroxy groups of 1 to 35%. Likewise, HPC can be preferably used, especially with a weight-average molecular weight of 50,000 to 100,000 g/mol. Also, polyethylene glycol with a weight-average molecular weight of 2,000 to 40,000 g/mol, especially from 3,500 to 25,000 g/mol, can be preferably used. Likewise, a polyethylene/polypropylene block copolymer can be preferably used, wherein the polyethylene content can be preferably 70 to 90 % by weight. The polyethylene/polypropylene block copolymer preferably has a weight-average molecular weight of 1,000 to 30,000 g/mol, more preferably from 3,000 to 15,000 g/mol. The weight average molecular weight can usually be determined by means of gel permeation chromatography.

In a preferred embodiment, the surface stabilizer (b) used can be a copolymer of vinylpyrrolidone and vinyl acetate, especially with a weight-average molecular weight of 45,000 to 75,000 g/mol. The copolymer can be characterised by the following structural formula (2):

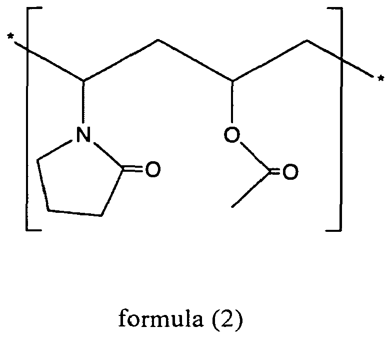

In a further preferred embodiment the surface stabilizer can be calcium phosphates, silicates or aluminosilicates. Especially preferred are silica, for example Aerosil^{®} 200, dicalcium phosphate (e.g. Dicafos AN) or magnesium aluminosilicate, for example Al₂O₃•MgO•1.7SiO₂•xH₂O (Neusilin^{®}).

In another preferred embodiment the pharmaceutical composition can further comprise one or more excipient(s) (c). Preferably, the excipient(s) (c) is selected from surfactants (c1), wicking agents (c2) and/or disintegrants (c3).

Surfactants (c1) are reported to be substances, lowering the interfacial tension between two phases, thus enabling or supporting the formation of dispersions or working as a solubilizer. Common surfactants can be alkyl sulfates (for example sodium lauryl sulfate), alkyltrimethylammonium salts, alcohol ethoxylates and the like.

Wicking agents (c2) are reported to be substances with the ability to draw a biological fluid (preferably water) into a solid, preferably by physisorption. Physisorption is defined as a form of adsorption, in which the solvent molecules can loosely adhere to surfaces of the wicking agent, preferably via van der Waals interaction between the surface of the wicking agent and the adsorbed fluid molecule (preferably water). Usually, a wicking agent can do this with or without swelling. Preferably, the wicking agent (c2) is a swelling wicking agent (c2). Usually, a non-swelling wicking agent, which attracts water, will ultimately have a volume that is essentially composed of the volume of the wicking agent and the volume of water attracted to it. Usually, a swelling wicking agent can have a volume that is essentially composed of the volume of the wicking agent, the volume of water attracted to it, and an additional volume created by steric and molecular forces.

Preferably, the wicking agent (c2) comprised in the present invention can create channels or pores in the granules. This can facilitate the channeling of water molecules through the granules, particularly by physisorption. Hence, the function of the wicking agent can be to carry water to surfaces inside the granules, thereby creating channels or a network of increased surface area.

Examples of wicking agents (c2) that may be used include, but are not limited to, microcrystalline cellulose, silicified microcrystalline cellulose, colloidal silicone dioxide, kaolin, titanium dioxide, fumed silicone dioxide, alumina, niacinamide, m-pyrol, bentonite, magnesium aluminum silicate, polyester, polyethylene, or mixtures thereof. Preferably, the wicking agents (c2) used in the pharmaceutical composition of the present invention can include cellulose and cellulose derivatives, such as silicified microcrystalline cellulose, colloidal silicone dioxide, and mixtures thereof. The silicified microcrystalline cellulose that is preferred is commercially available under the trade name Prosolv^{®}, having a silicone dioxide content from 1 to 3 wt.%, preferably of about 2 wt.%.

The wicking agent (c2) preferably can have an average particle size (D50) from 1 to 250 µm, more preferably from 20 to 200 µm, still more preferably from 30 to 150 µm, most preferably from 50 to 120 µm. As mentioned above, the average particle size can be determined by means of laser diffractometry.

Disintegrants (c3) are reported to be compounds which can enhance the ability of the intermediate to break into smaller fragments, when in contact with a liquid, preferably water. Preferred disintegrants are sodium carboxymethyl starch, crosslinked polyvinylpyrrolidone (Crospovidone), sodium carboxymethyl glycolate (for example Explotab^{®}), swelling polysaccharide, for example soy polysaccharide, carrageenan, agar, pectin, starch and derivates thereof, protein, for example formaldehyde-casein, sodium bicarbonate or mixtures thereof.

Generally, the pharmaceutical composition of the present invention can comprise the following amounts of components (a) ― (c):
5 to 90 wt.%, preferably 15 to 65 wt.%, more preferably 25 to 55 wt.% crizotinib (a),
5 to 90 wt.%, preferably 15 to 65 wt.%, more preferably 25 to 55 wt.% surface stabiliser (b),
0 to 20 wt.%, preferably 0.1 to 15 wt.%, more preferably 0.5 to 10 wt.% surfactant (c1),
0 to 60 wt.%, preferably 5 to 50 wt.%, more preferably 10 to 40 wt.% wicking agent (c2), and
0 to 60 wt.%, preferably 5 to 50 wt.%, more preferably 10 to 40 wt.% disintegrant (c3),
wherein the wt.% are based on the weight of the pharmaceutical composition.

Another subject of the present invention can relate to a method of preparing a pharmaceutical composition comprising the steps of
(i1) providing crizotinib and surface stabilizer (b), and optionally one or more excipient(s) (c),
(i2) melt processing the mixture from step (i1) to an intermediate, optionally cooling off, and
(i3) optionally granulating the intermediate from step (i2).

Generally, in step (i1) crizotinib, preferably crystalline crizotinib, can be present in an amount of 1 to 70 wt.%, preferably 4 to 40 wt.%, more preferably 5 to 35 wt.%, and particularly preferred between 6 and 30 wt.%, based on the total weight of the mixture resulting from step (i1).

Generally, in step (i1), surface stabilizer (b) can be present in an amount of 1 to 98 wt.%, preferably 15 to 85 wt.%, more preferably 20 to 70 wt.%, and particularly preferred between 30 and 60 wt.%, based on the total weight of the mixture resulting from step (i1).

Especially preferred surface stabilisers (b) in these embodiments are polyvinyl caprolactam-polyvinyl acetate-poly-ethylene glycol graft copolymers (e.g. Soluplus^{®}) or vinylpyrrolidone-vinylacetate copolymers (e.g. Kollidon VA64^{®}).

Generally, in step (i1), surfactants (c1) can be present in an amount of 0 to 3 wt.%, preferably 0.1 to 2 wt.%, more preferably 0.15 to 1.5 wt.%, based on the total weight of the mixture resulting from step (i1).

Generally, in step (i1), wicking agent (c2) can be present in an amount of 0 to 80 wt.%, preferably 5 to 70 wt.%, more preferably 10 to 65 wt.%, and particularly preferred between 15 and 50 wt.%, based on the total weight of the mixture resulting from step (i1).

Generally, in step (i1), disintegrant (c3) can be present in an amount of 0 to 45 wt.%, preferably 3 to 40 wt.%, more preferably 5 to 35 wt.%, and particularly preferred between 7 and 30 wt.%, based on the total weight of the mixture resulting from step (i1).

In another preferred embodiment, the provision of (i1) can be carried out with conventional mixing devices, e.g. in a free fall mixer like Turbula^{®} T 10B (Bachofen AG, Switzerland). Mixing can be carried out, e.g., for 1 minute to 1 hour, preferably for 5 to 30 minutes.

In step (i2) the mixture resulting from step (i1) is molten. In a preferred embodiment the melting conditions can be preferably chosen such that it is assured that crizotinib is obtained in a non-crystalline form.

The specific melting conditions can depend on compounds (a), (b) and optionally excipient(s) (c). Usually, temperatures from 40 °C to 200 °C, preferably from 60 °C to 180 °C are used. Preferably, crizotinib, the surface stabilizer (b) and the optionally excipient (s) (c) in their respective ratios may be chosen to achieve an eutectic mixture. Thus, the need of high temperatures for melting can be decreased, thereby optimizing energy demand.

In another embodiment, the cooling off step can be conducted under cooling conditions chosen such that non-crystalline crizotinib remains in a non-crystalline form. Non-crystalline crizotinib can be detected by XRD or DSC. Optionally, in step (i3) the molten mixture resulting from step (i2) is granulated, either in the molten state or after having cooled off.

Step (i3) of granulating the pharmaceutical composition can be carried out, for example, by an extrusion process. Hence, steps (i2) and (i3) preferably can be regarded as melt-extrusion processes. Generally, the extrusion process should be capable of providing essentially spherical particles. Suitable extruders are, for example, screw-feed extruders (axial or endplate, dome and radial) or gravity extruders (cylinder roll, gear roll or radial). Screw-feed extruders are preferred.

The granulation can also, for example, be carried out by a - preferably heatable - High-Shear-Mixer (e.g. Diosna^{®} P1/6). In this case, steps (i1), (i2) and (i3) can be regarded as one process with different sequences of special parameters. The first sequence can be step (i1) without heating, the second sequence can be a mixture of step (i1) and step (i2) with heating, sequence three can include parts of step (i2) and step (i3). Preferred parameters of the sequences can be dependent upon the chosen components (a), (b) and optionally excipient(s) (c).

In a preferred embodiment the granulation can be carried out with a melt screw extruder (e.g. Thermo Fisher^{®} Eurolab 16), wherein steps (i1) and (i3) can be unified in one continuous process. Generally, a temperature gradient can be applied, preferably between 70-200 °C.

In a preferred embodiment the granulation conditions in step (i3) are chosen such that the resulting granulated pharmaceutical composition can comprise an average particle size (D50) of 10 to 500 µm, more preferably of 50 to 250 µm, further more preferably of 60 to 200 µm, most preferably of 90 to 180 µm.

The bulk density of the granulated pharmaceutical composition made by the process of the present invention generally can range from 0.2 to 0.85 g/ml, preferably from 0.25 to 0.85 g/ml, more preferably from 0.3 to 0.75 g/ml.

The granulated pharmaceutical composition resulting from step (i3) of the present invention preferably possesses Hausner ratios in the range of 1.02 to 1.6, preferably of 1.08 to 1.4, more preferably between 1.1 to 1.3. The Hausner ratio is the ratio of tapped density to bulk density. Bulk density and tapped density can be determined according to USP 24, Test 616 "Bulk Density and Tapped Density".

In a further preferred method, the invention relates to a spray-drying process for preparing the pharmaceutical composition of the invention, comprising the steps of
(j1) dissolving crizotinib, the surface stabilizer (b) and optionally one or more excipient(s) (c) in a solvent or mixture of solvents, and
(j2) spray-drying the solution from step (j1).

In step (j1), crizotinib, surface stabilizer (b) and optionally one or more excipient(s) (c) are dissolved, preferably completely dissolved, in a solvent or mixture of solvents. It is preferable to use crystalline crizotinib.

Suitable solvents are, for example, water, alcohol (e.g. methanol, ethanol, isopropanol), acetone, butanol, ethyl acetate, heptane, pentanol or mixtures thereof. Preferably, an ethanol/water mixture is used.

Suitable surface stabilizers (b) in this first method are, in particular, modified celluloses, such as HPMC (preferably with a weight-average molecular weight of 20,000 to 90,000 g/mol), polyvinyl pyrrolidone and copolymers thereof (preferably with a weight-average molecular weight of 20,000 to 70,000 g/mol) and sugar alcohols, such as isomalt and sorbitol. As described above, the weight-average molecular weight is determined by gel permeation chromatography.

In the subsequent step (j2), the solution from step (j1) is spray-dried. The spray-drying is usually carried out in a spray tower. As an example, a Büchi B-191 is suitable (Büchi Labortechnik GmbH, Germany). Preferably, an inlet temperature of 100 °C to 150 °C is chosen. The amount of air is, for example, 500 to 700 liters/hour, and the aspirator preferably is set to 80-100%.

The process conditions in this second method are preferably selected such that the resulting particles of the pharmaceutical composition have a volume-average particle diameter (D50) of 1 to 250 µm, more preferably 5 to 150 µm, especially 10 to 100 µm.

In a further preferred embodiment, the invention can relate to a milling process, i.e. a method of preparing the pharmaceutical composition of the invention, comprising the steps of
(k1) mixing crizotinib, surface stabiliser (b) and optionally one or more excipient(s) (c), and
(k2) milling the mixture from step (11) to an intermediate, whereby the milling conditions are selected such that it is assured that non-crystalline crizotinib is obtained, and
(k3) optionally granulating the intermediate from step (12).

Preferably, crystalline crizotinib, surface stabiliser (b) and optionally one or more excipient(s) (c) are mixed in step (k1). The mixture is milled in step (k2). The mixing may take place before or even during the milling, i.e. steps (k1) and (k2) may be performed simultaneously.

The milling conditions are selected such that it is assured that non-crystalline crizotinib is obtained. The milling is generally performed in conventional milling apparatuses, preferably in a ball mill, such as a Retsch^{®} PM 100.

The milling time is usually 10 minutes to 10 hours, preferably 30 minutes to 8 hours, more preferably 2 hours to 6 hours.

Suitable surface stabilisers (b) in this embodiment can be, in particular, modified celluloses, such as HPMC, sugar alcohols, such as mannitol and sorbitol, and polyethylene glycol, in particular, polyethylene glycol with a molecular weight of 2,000 to 10,000 g/mol or magnesium alumino-metasilicate (e.g. Neusilin^{®}). Magnesium alumino-metasilicate is preferably used.

The process conditions in this embodiment are preferably selected such that the resulting particles of the pharmaceutical composition have an average particle size (D50) of 5 to 250 µm, more preferably 10 to 150 µm, especially 20 to 80 µm. Alternatively, the average particle size (D50) of the pharmaceutical composition is 20 to 150 µm, more preferably 50 to 100 µm.

Step (k3) of optionally granulating the pharmaceutical composition from step (k2) can be performed, for example, by "slugging", using a large heavy-duty rotary press and breaking up the slugs to granules with a hammer mill or by roller compaction, using for example roller compactors from Powtec or Alexanderwerk.

In another preferred embodiment, the pharmaceutical composition is obtained by one of the above-mentioned methods. Preferably the pharmaceutical composition can be available in a suitable form for further processing, for example as granules.

A further preferred embodiment of the present invention can be an oral dosage form comprising a pharmaceutical composition as described herein. The particles of the pharmaceutical composition, preferably granules, can preferably be regarded as an "intermediate pharmaceutical composition", which, preferably, is suitable for being further processed to an oral dosage form (which represents a "ready for use pharmaceutical composition"). The particles of the pharmaceutical composition can also be referred to as "internal phase" of the resulting oral dosage form. Hence, a further subject of the present invention is a pharmaceutical dosage form, preferably a solid pharmaceutical dosage form, in particular a solid oral dosage form, comprising the pharmaceutical composition of the present invention.

In a preferred embodiment of the present invention the oral dosage form can further comprise one or more excipient(s) (c'). The excipients (c') can be selected e.g. from surfactants (c1'), wicking agents (c2'), disintegrants (c3'), fillers (c4'), lubricants (c5') and glidants (c6').

All explanations given above for surfactant (c1) also apply to surfactant (c1'). Components (c1) and (c1') can be the same or different surfactants. Similar, all explanations given above for wicking agent (c2) also apply to wicking agent (c2'). Components (c2) and (c2') can be the same or different wicking agents. Furthermore the same considerations can be applied to disintegrants. Thus, components (c3) and (c3') can be the same or different disintegrants. These excipients can be referred to as "external phase" of the "ready for use pharmaceutical composition", preferably of the oral dosage form.

The oral dosage form can be present in form of a capsule or a solid tablet, preferably in form of a solid tablet.

Fillers (c4') or diluents can be used to increase the bulk volume and weight of a low-dose drug to a limit, at which a pharmaceutical dosage can be formed. Fillers may fulfil several requirements, such as being chemically inert, non-hygroscopic, biocompatible, easily processable and possessing good biopharmaceutical properties. Examples of fillers are lactose, sucrose, glucose, mannitol, calcium carbonate, cellulose and others.

Lubricants (c5') can be used in order to reduce sliding friction. In particular, the intention is to reduce the sliding friction occurring during tablet pressing between the moving up and down in the die and the die wall, on the one hand, and between the edge of the tablet and the die wall, on the other hand. The lubricant can be preferably a stearate or fatty acid, more preferably an earth alkali metal stearate, such as magnesium stearate. The lubricant can be suitably present in an amount of 0 to 3 wt.%, preferably of about 0.1 to 1.0 wt.% of the total weight of the final pharmaceutical composition.

Glidants (c6') can be used to improve the flowability. Traditionally, talc was used as glidant, but it is nowadays nearly fully replaced by colloidal silica (for example Aerosil^{®}). Preferably, the glidant agent can be present in an amount of up to 3 wt.%, Preferably, the silica has a specific surface area of 50 to 400 m²/g, measured by gas adsorption according to Ph. Eur., 6.0, chapter 2.9.26, multipoint method, volumetric determination.

It lies in the nature of pharmaceutical excipients that they sometimes can perform more than one function in a pharmaceutical formulation. Therefore, some pharmaceutically acceptable ingredients may function as pharmaceutical excipient (c) as well as surface stabilizer (b), i.e. the fact that an ingredient is used e.g. as a surfactant, a wicking agent or a disintegrant does not mean that it cannot also be acting as a surface stabilizer (b). For example, sodium lauryl sulphate in an oral dosage form may both act as a surface stabilizer and as a surfactant.

However, in the context of this invention, in order to provide an unambiguous delimitation, the fiction will therefore preferably apply that a substance, which is used as a particular excipient, is not simultaneously also used as a further pharmaceutical excipient. For example, microcrystalline cellulose ― if used as a wicking agent (c2) ― is not also used as for example a disintegrant (even though microcrystalline cellulose also exhibits a certain disintegrating effect).

A further subject of the present invention can be a process for an oral dosage form, comprising the steps of
(11) providing crizotinib and surface stabiliser (b) and optionally one or more excipient(s) (c),
(12) processing the mixture from step (11) to a pharmaceutical composition, in which non-crystalline crizotinib is obtained,
(13) optionally granulating the intermediate from step (12),
(14) mixing the pharmaceutical composition from step (12) or the granules from step (13) and optionally one or more excipient(s) (c'), and
(15) processing the mixture from step (14) into an oral dosage form; and
(16) optionally film-coating the dosage form.

In a preferred embodiment the features of the process steps (11), (12) and optionally (13) can correspond to the respective process steps of the methods for producing the pharmaceutical composition of the present invention, which have been described herein above.

In a preferred embodiment, step (14) can be characterized by mixing the pharmaceutical composition from step (12) or the granules resulting from step (13) with one or further excipient(s) (c'). The mixing (14) can be carried out with conventional mixing devices, e.g. in a free fall mixer like Turbula^{®} T 10B (Bachofen AG, Switzerland). Mixing can be carried out for example for 1 minute to 1 hour, preferably for 5 to 30 minutes.

In another preferred embodiment, the process for producing an oral dosage form according to the present invention can be characterized by a specific ratio of the amount of wicking agent in the internal phase (c2) to wicking agent in the external phase (c2'). In a preferred embodiment the weight ratio of wicking agent (c2) to wicking agent (c2') can be from 1:6 to 3:1, more preferably from 1:3 to 3:2.

In another preferred embodiment the process for producing an oral dosage form according to the present invention can be characterized by a specific ratio of the amount of disintegrant in the internal phase (c3) to disintegrant in the external phase (c3'). In a preferred embodiment the weight ratio of disintegrant (c3) to disintegrant (c3') can be from 1:5 to 5:2, more preferably from 2:5 to 2:1.

In a further embodiment the weight ratio of the total amount of wicking agent ((c2)+(c2')) to total amount of disintegrant ((c3)+(c3')) can preferably be from 5:1 to 2:3, more preferably from 3:1 to 1:1.

In addition, in the mixing step (m4) preferably one or more further excipient(s), such as fillers (c4'), lubricants (c5') and glidants (c6'), can be used. Regarding the above mentioned pharmaceutically acceptable excipients, the application generally refers to "Lexikon der Hilfsstoffe für Pharmazie, Kosmetik und angrenzende Gebiete", edited by H. P. Fiedler, 5th Edition, Editon Cantor, Aulendorf and earlier editions, and "Hand-book of Pharmaceutical Excipients", third edition, edited by Arthur H. Kibbe, American Pharmaceutical Association, Washington, USA, and Pharmaceutical Press, London.

In another preferred embodiment, step (15) of optionally processing the mixture of step (14) into an oral dosage form preferably can comprise, for example, compressing the mixture of step (14) into tablets or filling mixture of step (14) into capsules. Preferably the mixture can be compressed into tablets.

Compressing, preferably direct compressing, the mixture of step (15) into tablets can be carried out by compressing said formulation on a rotary press, e.g. on a Fette^{®} (Fette GmbH, Germany) or a Riva^{®} Piccola (Riva, Argentina). The compression force usually ranges from 1 to 50 kN, preferably 3 to 40 kN. The resulting tablets can have a hardness of 30 to 400 N, more preferred 50 to 325 N, still more preferred from 75 to 300 N, in particular from 85 to 275 N, wherein the hardness is measured according to Ph. Eur., 6.0, chapter 2.9.8. In step (16) the tablet can optionally be film-coated.

In addition, the resulting tablets preferably can have a friability of less than 5 %, particularly preferably less than 2 %, especially less than 1 %. The friability is determined in accordance with Ph. Eur., 6.0, chapter 2.9.7. The friability of tablets generally refers to tablets without coating.

Finally, the tablets of the invention preferably can have a "content uniformity" of 90 to 110 %, preferably 95 to 105 %, especially 98 to 102 % of the average content. The "content uniformity" is determined in accordance with Ph. Eur., 6.0, chapter 2.9.6. This means that each of twenty individual samples of the dosage form has a crizotinib content of between 90% and 110%, preferably 95% to 105%, even more preferably 98% to 102% of the average content of those twenty individual samples.

In another embodiment, processing the mixture of step (14) to an oral dosage form can be done by or filling the mixture of step (14) into capsules, preferably hard gelatine capsules. For this filling of the mixture of step (14) into capsules, dependent dosing systems (for example an auger) or preferably independent dosing systems (for example MG2, Matic (IMA)) can be used.

Generally, the dosage form of the present invention can comprise the following amounts of components (a), (b), (c) and (c'):
5 to 80 wt.%, preferably 15 to 60 wt.%, more preferably 20 to 50 wt.% crizotinib (a),
2 to 80 wt.%, preferably 5 to 50 wt.%, more preferably 10 to 40 wt.% surface stabiliser (b),
0 to 20 wt.%, preferably 0.1 to 15 wt.%, more preferably 0.5 to 5wt.% surfactant (c1/c1'),
0 to 60 wt.%, preferably 2 to 40 wt.%, more preferably 5 to 30 wt.% wicking agent (c2/c2'), and
0 to 50 wt.%, preferably 4 to 35 wt.%, more preferably 5 to 25 wt.% disintegrant (c3/c3'),
0 to 10 wt.%, preferably 5 to 50 wt.%, more preferably 10 to 40 wt.% filler (c4'),
0 to 5 wt.%, preferably 0.1 to 3 wt.%, more preferably 0.2 to 2.0 wt.% lubricant (c5'); and
0 to 5 wt.%, preferably 0.1 to 2 wt.%, more preferably 0.2 to 2.0 wt.% glidant (c6'),
wherein the wt.% are based on the weight of the dosage form.

Preferably, the dosage form of the present invention can comprise an inner phase comprising components (a), (b), (c) and an outer phase, comprising one or more of components (c1'-c6'), in particular the inner phase comprises 5 to 80 wt.%, preferably 15 to 60 wt.%, more preferably 20 to 50 wt.% crizotinib, preferably non-crystalline crizotinib (a),
2 to 80 wt.%, preferably 5 to 50 wt.%, more preferably 10 to 40 wt.% surface stabiliser (b),
0 to 20 wt.%, preferably 0.1 to 15 wt.%, more preferably 0.5 to 5 wt.% surfactant (c1),
0 to 50 wt.%, preferably 1 to 40 wt.%, more preferably 5 to 25 wt.% wicking agent (c2), and
0 to 50 wt.%, preferably 2 to 30 wt.%, more preferably 3 to 20 wt.% disintegrant (c3),
and in particular the outer phase comprises
0 to 20 wt.%, preferably 0.1 to 10 wt.%, more preferably 0.2 to 2wt.% surfactant (c1'),
0 to 40 wt.%, preferably 1 to 30 wt.%, more preferably 2 to 20 wt.% wicking agent (c2'), and
0 to 30 wt.%, preferably 1 to 20 wt.%, more preferably 3 to 15 wt.% disintegrant (c3'),
0 to 50 wt.%, preferably 1 to 30 wt.%, more preferably 5 to 25 wt.% filler (c4'),
0 to 5 wt.%, preferably 0.1 to 3 wt.%, more preferably 0.2 to 2.0 wt.% lubricant (c5'); and
0 to 5 wt.%, preferably 0.1 to 2 wt.%, more preferably 0.2 to 2.0 wt.% glidant (c6'),
wherein the wt.% are based on the weight of the dosage form.

When treating the diseases which are indicated for the active agent or the combination of active agents in the oral dosage forms of the invention, satisfactory results are usually obtained if the active agent crizotinib contained is administered in a daily dose of 50 to 1000 mg, preferably 75 to 750 mg, more preferably 100 to 500 mg and particularly 150 to 300 mg. In the same doses, applications less than once a day are possible, such as every two, three or four days, for example in a delayed-release formulation. The dosing regimen may be varied within or even outside this frame in order to achieve the optimum treatment results.

The oral dosage forms of the invention are usually characterised by a release and absorption that lead to advantageous figures for the AUC *("area under curve"),* the area under the curve of the plasma level 0 to 48 hours after peroral administration), advantageous figures for the Cₘₐₓ (maximum plasma level) and advantageous figures for the Tₘₐₓ (time when the maximum plasma level is reached after peroral administration).

In a preferred embodiment, the single-dose peroral administration of the oral dosage forms of the invention to a human patient can lead to a plasma level profile characterised by a Tₘₐₓ regarding the active agent crizotinib of about 0.5 to 3 hours.

In a preferred embodiment, the single-dose peroral administration of the oral dosage forms of the invention to a human patient can lead to a plasma level profile characterised by a Cₘₐₓ regarding the active agent crizotinib of about 40 to 120 ng/ml, preferably 50 to 100 ng/ml.

In a preferred embodiment, the single-dose peroral administration of the oral dosage forms of the invention to a human patient can lead to a plasma level profile characterised by an AUC regarding the active agent crizotinib of about 800 to 2,500 ng×h/ml, preferably 1,000 to 2,000 ngxh/ml.

The above-mentioned plasma level figures are preferably average values, obtainable by examining blood samples from a group of 10 candidates (with an average body weight of 70 kg), the corresponding blood samples being taken 0, 1, 3, 4, 6, 8, 24 and 48 hours after the peroral administration of the formulation of the invention. The plasma level figures can preferably be determined by means of appropriate HPLC-MSMS methods. The AUC as described herein is the infinite AUC after a single dose and can be calculated by, for example, using a computer program such as the Microsoft Excel program.

In a preferred embodiment, the oral dosage forms of the invention can be used to treat non-small cell lung carcinoma.

The subject matter of the invention is thus also an oral dosage form, such as a tablet, containing 50 to 500 mg, preferably 200 to 250 mg crizotinib, preferably non-crystalline crizotinib, the dosage form having a content uniformity of 95 to 105 %, and wherein the administration regarding the active agent crizotinib leads to a Tₘₐₓ of 0.5 to 3 hours, a Cₘₐₓ of 40 to 120 ng/ml, more preferably 50 to 100 ng/ml and an AUC of about 800 to 2,500 ng×h/ml. Preferably, the dosage form is a tablet having a hardness of 50 to 250 N and a friability of less than 5 %. In the tablet of the invention, crizotinib preferably is present in the form of the pharmaceutical composition of the invention.

In a preferred embodiment the composition and/ or the dosage form according to the invention provides an immediate release ("IR") of crizotinib. This means that the release profile of the dosage forms of the invention according to USP method (paddle, 900 ml, 0.1 n HCl, 75 rpm, 37 °C) after 10 minutes usually indicates a content release of at least 75 %, preferably at least 85 %, especially at least 90 %.

The dosage form of the invention tablets may be a tablet, which can be swallowed unchewed (non-film-coated or preferably film-coated).

In a preferred embodiment, the tablet of the present application can be film-coated. For this purpose, methods known in the art for film-coating a tablet may be employed.

Generally, film-coatings can be prepared by using cellulose derivatives, poly(meth)acrylate, polyvinyl pyrrolidone, polyvinyl acetate phthalate, and/or shellac or natural rubbers such as carrageenan.

In a preferred embodiment of the present invention the film-coating can be a film-coating essentially without affecting the release of the active agent.

Preferred examples of film-coatings, which do not affect the release of the active ingredient, can be those including poly(meth)acrylate, methylcellulose (MC), hydroxypropyl methylcellulose (HPMC), hydroxypropyl cellulose (HPC), hydroxyethyl cellulose (HEC), polyvinyl pyrrolidone (PVP) and mixtures thereof. These polymers can have an weight average molecular weight of 10,000 to 150,000 g/mol.

In an alternative preferred embodiment, the film-coating can affect the release of the active agent. Examples for film-coating affecting the release of the active agent are gastric juice resistant film-coatings and retard coatings.

In gastric juice resistant coatings the solubility depends on the pH of the surrounding.

Examples of gastric juice 'resistant' coatings can comprise cellulose acetate phthalate (CAP), hydroxypropyl methylcellulose phthalate (HPMCP) and polyvinyl acetate phthalate (PVAP). Surprisingly, it has been found that these coatings provide a superior gastrointestinal adverse effect profile.

Retard coatings are usually non-soluble (preferably having a water solubility at 25 °C of less than 10 mg/ml).

Examples of retard coatings can comprise ethyl cellulose (EC, commercially available e.g. as Surelease^{®}) and poly(meth)acrylate (commercially available e.g. as Eudragit^{®} RL or RS and L/S).

Further the coating can be free from active ingredient. However, it is also possible that the coating can contain an active ingredient (crizotinib). In such a case, this amount of active ingredient would function as an initial dose. In such a case, the coating preferably can comprise 1 to 45 wt.%, preferably 5 to 35 wt.%, most preferably 10 to 30 wt.% of crizotinib, based on the total amount of crizotinib contained in the tablet.

In case the film coating does not contain crizotinib (which is preferred), it can have a thickness of 2 µm to 100 µm, preferably from 20 to 60 µm. In case of a coating containing crizotinib, the thickness of the coating is usually 10 µm to 200 µm, preferably from 50 to 125 µm.

### EXAMPLES

### Melt Extrusion

Crizotinib free base and polymer are mixed together through a screen mesh size 1000 µm. The premix is blended in a tumble blender (Bachofen Turbula^{®} T 10B) for 15 min. The premix is extruded on a Thermo Fisher Eurolab 16 twin screw extruder using the following temperature profile:

| | |
|---|---|
| Input | 20 - 30 °C |
| zone 2 | 70 - 80 °C |
| zone 3 | 110 - 120 °C |
| zone 4 | 140 - 160 °C |
| zone 5 | 170 - 200 °C |
| zone 6 | 170 - 200 °C |
| zone 7 | 140 - 160 °C |
| zone 8 | 120 - 140 °C |
| zone 9 | 100 - 130 °C |
| "die" | 90 - 110 °C |

Extrusion of crizotinib and Neusilin is performed without "die".

The following extrudates are prepared:
Extrudate 1) API : Kollidon^{®} VA 64 2 : 1
Extrudate 2a) API : Soluplus^{®} 1.5 : 1
Extrudate 2b) API : Soluplus^{®} 1 : 2
Extrudate 3a) API : Kollidon^{®} 30 1 : 1
Extrudate 3b) API : Kollidon^{®} 30 1 : 2
Extrudate 4) API : Neusilin^{®} US2 3 : 1

The extrudates are allowed to cool and are then granulated and milled (ITE CoMil^{®}) through a screen 800 µm mesh size. The pharmaceutical composition is then mixed with a disintegrant, a filler, flow enhancers and lubricant according to the tables below, and the final mixture is compressed into tablets. Alternatively, the final mixture can be filled into hard gelatine capsules.

### Final Tablet Formulation:

| Compound | amount [mg] | amount [%] |
|---|---|---|
| Extrudate 1 | 375.0 (250 mg active ingredient) | 70.0 |
| Lactose monohydrate | 120.54 | 22.5 |
| Crospovidone | 32.14 | 6.0 |
| Silica | 2.68 | 0.5 |
| Magnesium stearate | 5.36 | 1.0 |
| **TOTAL** | **535.72** | **100** |

| Compound | amount [mg] | amount [%] |
|---|---|---|
| Extrudate 2a | 416.67 (250 mg active ingredient) | 80.0 |
| Dicalcium phosphate | 67.71 | 13.0 |
| Crospovidone | 28.65 | 5.5 |
| Silica | 2.60 | 0.5 |
| Magnesium stearate | 5.21 | 1.0 |
| **TOTAL** | **520.84** | **100** |

| Compound | amount [mg] | amount [%] |
|---|---|---|
| Extrudate 2b | 600.0 (200 mg active ingredient) | 85.0 |
| Dicalcium phosphate | 70.59 | 10.0 |
| Crospovidone | 24.70 | 3.5 |
| Silica | 3.53 | 0.5 |
| Magnesium stearate | 7.06 | 1.0 |
| **TOTAL** | **705.88** | **100** |

| Compound | amount [mg] | amount [%] |
|---|---|---|
| Extrudate 3a | 500.0 (250 mg active ingredient) | 85.0 |
| Dicalcium phosphate | 58.82 | 10.0 |
| Crospovidone | 20.59 | 3.5 |
| Silica | 2.94 | 0.5 |
| Magnesium stearate | 5.88 | 1.0 |
| **TOTAL** | **588.23** | **100** |

| Compound | amount [mg] | amount [%] |
|---|---|---|
| Extrudate 3b | 600.0 (200 mg active ingredient) | 85.0 |
| Dicalcium phosphate | 70.59 | 10.0 |
| Crospovidone | 24.70 | 3.5 |
| Silica | 3.53 | 0.5 |
| Magnesium stearate | 7.06 | 1.0 |
| **TOTAL** | **705.88** | **100** |

| Compound | amount [mg] | amount [%] |
|---|---|---|
| Extrudate 4 | 333.33 (250 mg active ingredient) | 70.0 |
| Lactose monohydrate | 107.14 | 22.5 |
| Crospovidone | 28.57 | 6.0 |
| Silica | 2.38 | 0.5 |
| Magnesium stearate | 4.76 | 1.0 |
| **TOTAL** | **476.18** | **100** |

### Spray-drying:

10 g crizotinib free base is dissolved in an adequate amount of ethanol. 5 g HPMC are added and the mixture is stirred together for 15 min. The solution is spray-dried on a Büchi^{®} B 290. The spray-dried material is mixed together with the further excipients of the table below and compressed into tablets or, alternatively, filled into capsules.

| Compound | amount [mg] | amount [%] |
|---|---|---|
| Spray dried material | 375.0 (250 mg active ingredient) | 70.0 |
| Lactose monohydrate | 120.54 | 22.5 |
| Crospovidone | 32.14 | 6.0 |
| Silica | 2.68 | 0.5 |
| Magnesium stearate | 5.36 | 1.0 |
| **TOTAL** | **535.72** | **100** |

### Co-milling:

Crizotinib and magnesium alumino-metasilicate (Neusilin^{®}) are mixed together and milled in a Retsch^{®} PM 100 mill for 8 h. The mixture is sieved (mesh size 800 µm) and mixed together with the sieved remaining excipients as listed in the table below. The mixture is compressed into tablets or, alternatively, filled into capsules.

| Compound | amount [mg] | amount [%] |
|---|---|---|
| API : Neusilin^{®} US2 3 : 1 | 333.33 | 70.0 |
| Lactose monohydrate | 107.14 | 22.5 |
| Crospovidone | 28.57 | 6.0 |
| Silica | 2.38 | 0.5 |
| Magnesium stearate | 4.76 | 1.0 |
| **TOTAL** | **476.18** | **100** |

### Pre-trials for formulation by DSC:

Equipment: Mettler Toledo DSC1_399 cell with IntraCooler, 100 µl aluminium crucible; measurement parameters: weigh about 20-30 mg of a binary mixture of crizotinib and the corresponding excipient into a sample container, close the container and place it in the heating chamber.

The measurement is carried out under nitrogen atmosphere, the crucible is punched prior to analysis. The parameters are as follows:

| | |
|---|---|
| 1^{st} segment: | -50 °C, holding time 1 min |
| 2^{nd} segment: | heating -50 °C ― 110 °C, heating rate 10 °C min⁻¹ |
| 3^{rd} segment: | 110 °C, holding time 5 min |
| 4^{th} segment: | cooling 110 °C - -50 °C, cooling rate -50 °C min⁻¹ |
| 5^{th} segment: | -50 °C, holding time 1 min |
| 6^{th} segment: | -50 °C - 230 °C, heating rate 10°C min⁻¹ |

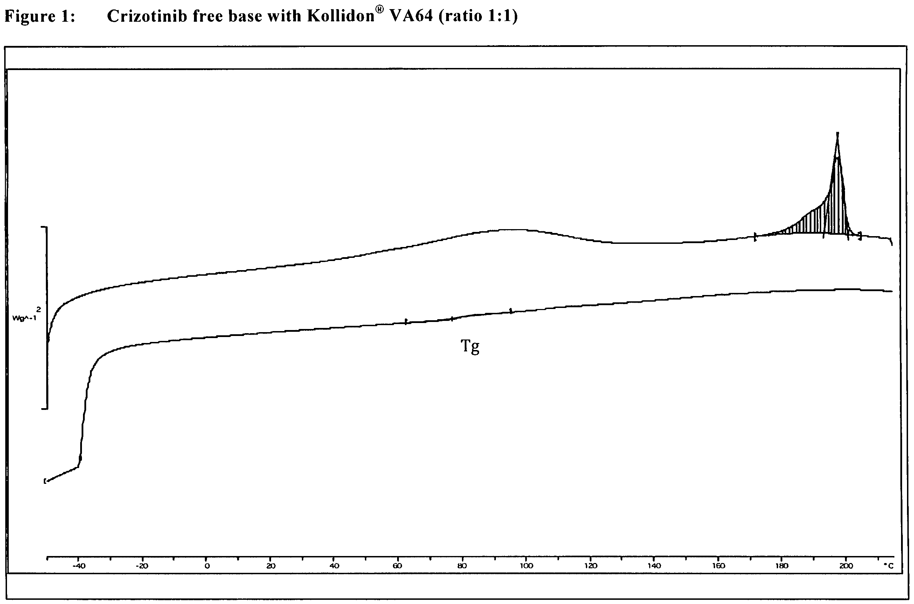

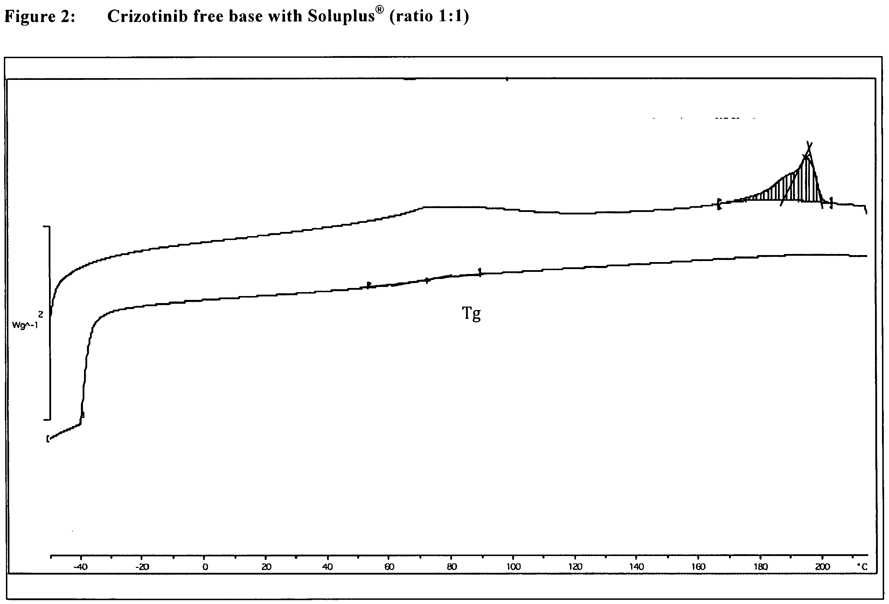

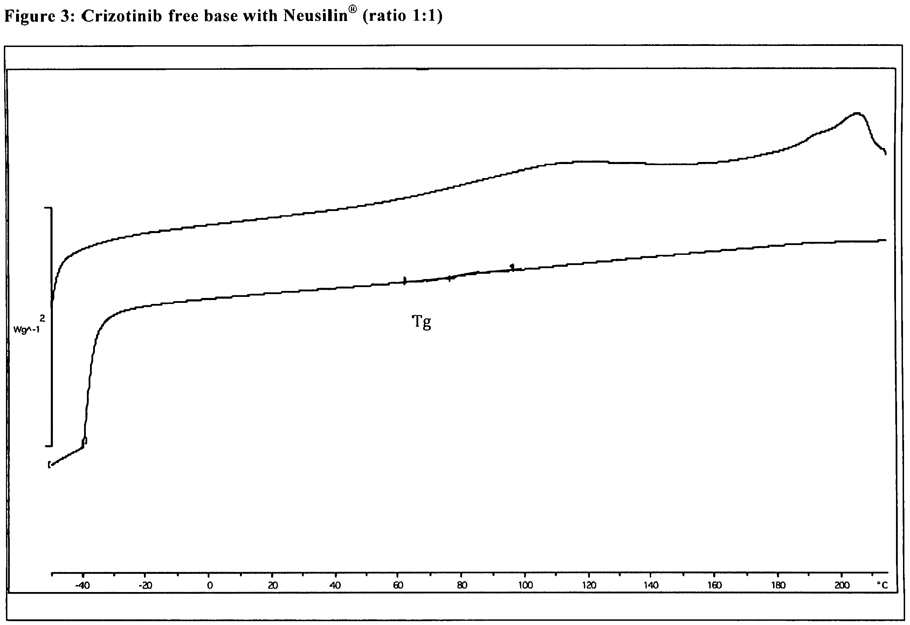

## Claims

1. A pharmaceutical composition comprising non-crystalline crizotinib (a) and surface stabiliser (b).

2. A pharmaceutical composition according to claim 1, wherein the weight ratio of non-crystalline crizotinib (a) to surface stabiliser (b) is from 1: 10 to 10: 1.

3. A pharmaceutical composition according to claim 1 or 2, wherein the surface stabiliser (b) is a substance inhibiting the recrystallisation of non-crystalline to crystalline crizotinib such that no more than 20% present at the time of manufacture is transformed into crystalline crizotinib upon storage for three months at 45 °C and 75% relative humidity.

4. A pharmaceutical composition according to anyone of the previous claims, wherein the surface stabiliser (b) is a polymer, preferably having a glass transition temperature (Tg) of more than 25 °C or a non-polymeric compound selected from fats, waxes, sugar alcohols and disaccharides.

5. A pharmaceutical composition according to anyone of the previous claims, wherein the non-crystalline crizotinib (a) is present in amorphous form.

6. A pharmaceutical composition according anyone of the previous claims, further comprising one ore more excipient(s) (c), wherein the excipient(s) (c) is/are selected from surfactants (c1), wicking agents (c2) and/or disintegrants (c3).

7. A method of preparing a pharmaceutical composition according to anyone of the previous claims, comprising the steps of
(i1) providing crizotinib and surface stabiliser (b) and optionally one or more excipient(s) (c),
(i2) melt processing the mixture from step (i1) to a pharmaceutical composition, and
(i3) optionally granulating the pharmaceutical composition from step (i2).

8. A method of preparing a pharmaceutical composition according to claim 7, wherein the conditions of the melting process (i2) are chosen such that they assure that crizotinib is obtained in a non-crystalline form.

9. A method of preparing a pharmaceutical composition according to claims 1 to 6 comprising the steps of
(j1) dissolving crizotinib, surface stabiliser (b) and optionally one or more excipient(s) (c) in a solvent or mixture of solvents,
(j2) spray-drying the solution from step (j1).

10. A method of preparing a pharmaceutical composition according to claims 1 to 6 comprising the steps of
(k1) mixing crizotinib, surface stabiliser (b) and optionally one or more excipient(s) (c) and
(k2) milling the mixture from step (11) to a pharmaceutical composition, whereby the milling conditions are selected such to assure that non-crystalline crizotinib is obtained, and
(k3) optionally granulating the pharmaceutical composition from step (12).

11. A pharmaceutical composition, obtainable by a method as claimed in anyone of claims 8 to 10.

12. An oral dosage form comprising a pharmaceutical composition according to claims 1 to 6 and 11.

13. An oral dosage form according to claim 12 comprising an inner phase and an outer phase, wherein the inner phase comprises (a), (b) and (c) and the outer phase comprises excipient(s) (c'), wherein excipient(s) (c') preferably are selected from surfactants (c1'), wicking agents (c2'), disintegrants (c3'), fillers (c4'), lubricants (c5') and glidants (c6').

14. Process of producing an oral dosage form according to any one of claims 12 to 13, comprising the steps of
(11) providing crizotinib and surface stabiliser (b) and optionally one or more excipient(s) (c),
(12) processing the mixture from step (11) to a pharmaceutical composition, in which non-crystalline crizotinib is obtained,
(13) optionally granulating the pharmaceutical composition from step (12),
(14) mixing the pharmaceutical composition from step (12) or the granules from step (13) with optionally one or more excipient(s) (c'), and
(15) processing the mixture from step (14) into an oral dosage form.

15. Oral dosage form containing 200 to 250 mg crizotinib, said dosage form having a content uniformity of 95 to 105%, and wherein a single-dose administration regarding the active agent crizotinib leads to a Tₘₐₓ of 0.5 to 3 hours, a Cₘₐₓ of 40 to 120 ng/ml and an AUC of about 800 to 2,500 ng×h/ml.
